(19) Europäisches Patentamt
European Patent Office
Office européen des brevets

(11) Veröffentlichungsnummer: **0 291 055 B1**

(12) # EUROPÄISCHE PATENTSCHRIFT

(45) Veröffentlichungstag der Patentschrift: **15.09.93**

(51) Int. Cl.$^5$: **C12N 15/57**, C12N 9/78, C12N 1/20, C12Q 1/34, //(C12N1/20,C12R1:19)

(21) Anmeldenummer: **88107636.8**

(22) Anmeldetag: **11.05.88**

Die Akte enthält technische Angaben, die nach dem Eingang der Anmeldung eingereicht wurden und die nicht in dieser Patentschrift enthalten sind.

(54) **Stabile Creatinamidinohydrolase-Mutanten.**

(30) Priorität: **12.05.87 DE 3715841**
**03.02.88 DE 3803175**

(43) Veröffentlichungstag der Anmeldung:
**17.11.88 Patentblatt 88/46**

(45) Bekanntmachung des Hinweises auf die Patenterteilung:
**15.09.93 Patentblatt 93/37**

(84) Benannte Vertragsstaaten:
**AT BE CH DE ES FR GB GR IT LI LU NL SE**

(56) Entgegenhaltungen:
**EP-A- 0 187 138**
**DE-A- 3 707 172**

**PATENT ABSTRACTS OF JAPAN, Band 6, Nr. 106 (C-108)[984], 16. Juni 1982; & JP-A-57 36 985 (TOYO BOSEKI K.K.) 27-02-1982**

**PROTEINS: STRUCTURE, FUNCTION AND GENETICS, Band 1, Nr. 4, 1986, Seiten 326-334, A.R. LISS, INC., New York, NY, US; P.N. BRYAN et al.: "Proteases of enhanced stability: Characterization of a thermostable variant of subtilisin"**

(73) Patentinhaber: **BOEHRINGER MANNHEIM GMBH**

**D-68298 Mannheim(DE)**

(72) Erfinder: **Schumacher, Günther, Dr. rer. nat.**
**Kapellenweg 20**
**D-8139 Bernried(DE)**
Erfinder: **Buckel, Peter, Dr. rer. nat.**
**Eichenstrasse 19**
**D-8139 Bernried(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**D-81635 München (DE)**

EP 0 291 055 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Mutanten der Creatinamidinohydrolase, welche stabiler sind als das Wildtypenzym, und deshalb besser zur enzymatischen Bestimmung des Creatiningehalts im Serum, Plasma oder Harn geeignet sind.

Das Enzym Creatinamidinohydrolase EC 3.5.3.3 findet industriell Anwendung zur Creatininbestimmung. Es wird u. a. in der klinischen Analytik für die Diagnose von Nierenerkrankungen verwendet, bei welchen im Serum oder Urin Creatiningehalte auftreten, die von denen des gesunden Organismus abweichen. Es sind Mikroorganismen bekannt, wie beispielsweise Pseudomonas-Arten, welche unter Induktion durch Creatin in der Lage sind, Creatinamidinohydrolase in einer die Aufarbeitung lohnenden Menge herzustellen, jedoch stellen die erzielbaren Ausbeuten und die Kosten der Isolierung des Enzyms immer noch einen limitierenden Faktor für die industrielle Anwendung des Enzyms dar. Wie in der deutschen Offenlegungsschrift 35 00 184 beschrieben ist, ist es gelungen, das für die Creatinamidinohydrolase kodierende Gen aus Pseudomonas putida zu isolieren und beispielsweise in das Plasmid pBR322 einzubringen. Nach Transformation eines Mikroorganismus der Gattung E. coli oder Pseudomonas putida ist es möglich, aus diesen konstitutiv Creatinamidinohydrolase zu gewinnen. Diese gentechnologische Herstellung der Creatinamidinohydrolase ist effektiver und leichter durchzuführen als die Isolierung aus einem induzierten, kein Plasmid enthaltenden Mikroorganismus. Dieses Verfahren hat jedoch den Nachteil, daß das daraus gewonnene Wildtypenzym nur beschränkte Detergenz- und thermische Stabilität aufweist und somit für den Einsatz im klinischen Testverfahren nicht optimal geeignet ist.

Aufgabe der vorliegenden Erfindung ist es, Creatinamidinohydrolase-Mutanten bereitzustellen, welche die geschilderten Nachteile des Standes der Technik nicht oder nur in geringerem Maße aufweisen. Erfindungsgemäß wird diese Aufgabe gelöst durch Creatinamidinohydrolase-Mutanten, welche die Reaktion

Creatin + $H_2O \rightarrow$ Sarcosin + Harnstoff

entsprechend dem Wildtypenzym katalysieren, und dadurch gekennzeichnet sind, daß gegenüber dem Wildtypenzym mindestens ein Aminosäureaustausch an der Position 109 stattgefunden hat. Dieser Austausch betrifft die Aminosäure Alanin, die durch Valin ersetzt wurde. Das so mutierte Enzym besitzt eine weit bessere Stabilität als das Wildtypenzym. Abgesehen von diesem Aminosäureaustausch können ohne Beeinträchtigung der enzymatischen Aktivität auch andere Mutationen auf Aminosäureebene vorliegen und dabei sogar eine noch höhere Stabilität erreicht werden. Ein bevorzugtes Enzym enthält außer dem Aminosäureaustausch an Position 109 einen weiteren Aminosäureaustausch.

Weitere Gegenstände der Erfindung sind die Plasmide pBT 109 und pBT 119. Diese enthalten das Creatinamidinohydrolasegen des Wildtyps, wobei in beiden Plasmiden jedoch auf DNA-Ebene bereits der Aminosäureaustausch an der Position 109 des Enzyms und darüberhinaus bei pBT 119 auch in einer weiteren Position, nämlich Position 355 in Form eines Wechsels von Val nach Meth durch entsprechenden Austausch der im Wildtypgen diese Aminosäuren codierenden Tripletts festgelegt ist. Durch Sequenzierung von pBT 119 wurde festgestellt, daß an Position 1063 der Sequenz im kodierenden Strang ein G durch A ersetzt ist ( G $\rightarrow$ A), so daß das Triplett GTG des Wildtyps in ATG umgewandelt ist. Ein bevorzugter Mikroorganismus der Gattung E. coli gemäß der Erfindung, E. coli, DSM 4105, enthält das Plasmid pBT 109, ein weiterer bevorzugter Mikroorganismus E. coli, DSM 4106 enthält erfindungsgemäß das Plasmid pBT 119. Weitere bevorzugte Mikroorganismen sind erfindungsgemäß solche der Gattung E. coli oder Pseudomonas putida, welche konstitutiv eine Creatinamidinohydrolase, welche die genannten Mutationen enthält, exprimieren.

Die Herstellung der erfindungsgemäßen mutierten Enzyme erfolgt, indem man nach an sich bekannten gentechnologischen Methoden eine rekombinante DNA in einem geeigneten Expressionssystem zur Expression bringt, welche ein Creatinamidinohydrolasegen enthält, welches die Sequenz des Wildtypgens im wesentlichen aufweist, aber mindestens in Position 326 des natürlichen Gens anstelle des Desoxyribonukleotids C ein T enthält. Vorzugsweise wird ein Gen exprimiert, bei dem außerdem an weiteren Positionen eine Base ausgetauscht wurde. Besonders bevorzugt liegt in Position 1063 anstelle von G ein A vor.

Bevorzugt wird als rekombinante DNA eines der Plasmide pBT 109, DSM 4108P oder pBT 119, DSM 4107P exprimiert. Als rekombinante DNA kann aber auch jegliche rekombinante DNA verwendet werden, welche die in der DE 35 00 184 A1 offenbarte DNA-Sequenz für das Wildtypenzym, oder ein nach dem genetischen Code für die gleiche Aminosäuresequenz kodierendes Äquivalent davon enthält, wobei jedoch an der Position 326 des natürlichen Gens anstelle von C das Desoxyribonukleotid T enthalten ist. In einer solchen rekombinanten DNA kann zusätzlich ein weiterer Basenaustausch, der zu einer weiteren Aminosäuremutation im Enzym führt, enthalten sein. Vorzugsweise ist dabei in Position 1063 G gegen A ausgetauscht.

Als Expressionssystem wird ein Mikroorganismus der Gattung E. coli oder Pseudomonas putida bevorzugt. Besonders bevorzugt verwendet man den Mikroorganismus E. coli ED 8654, DSM 2102 oder Pseudomonas putida, DSM 2106. Weitere erfindungsgemäße Herstellungsverfahren sind durch das Züchten der bevorzugten Mikroorganismen E. coli, DSM 4105 und E. coli, DSM 4106 gekennzeichnet.

Ein weiterer Gegenstand der Erfindung ist die Verwendung der erfindungsgemäßen, mutierten Creatinamidinohydrolasen, welche stabiler als das Wildtypenzym sind, zur Bestimmung des Creatiningehalts im Serum, Plasma oder Harn.

Untersuchungen der in der DE 35 00 184 A1 beschriebenen, klonierten Creatinamidinohydrolase haben ergeben, daß das Enzym den geschwindigkeitsbestimmenden Schritt in der Reaktionsabfolge

$$\text{Creatin} + H_2O \xrightarrow{\text{Creatinamidino hydrolase}} \text{Sarcosin} + \text{Harnstoff}$$

katalysiert. Eine Steigerung der Geschwindigkeit des Substratumsatzes ist unter den als optimal erarbeiteten Bedingungen durch Erhöhung der Enzymmenge nicht zu erzielen. Unter Testbedingungen zur Bestimmung des Creatiningehalts im Serum, Plasma oder Harn bei 37 °C weist das Enzym eine beschränkte Stabilität auf, was zu einer Verringerung des Substratumsatzes bei der oben erwähnten Temperatur führt. Die erfindungsgemäße Creatinamidinohydrolase, die eine Mutation der Aminosäure 109 des Wildtypenzyms von Alanin in Valin enthält, hat im Vergleich, unter verschiedenen Testbedingungen (Detergenzzusatz), bei ungefähr gleicher Ausgangsenzymaktivität bereits eine stark erhöhte Detergenzstabilität, wie die in Tabelle I wiedergegebenen Vergleichsversuchsergebnisse zeigen. Das Temperaturverhalten unter optimalen Bedingungen wird in Tab. II dargestellt.

Tabelle I

Enzymbestimmung bei 37 °C

| | Zeit (Minuten) | Creatinamidinohydrolase-Wildtyp (DE 35 00 184 A1) (DSM 3143) Vergleich | Creatinamidinohydrolase-Mutante (Aminosäure 109 = Val) (DSM 4105) gemäß Erfindung | Testbedingungen |
|---|---|---|---|---|
| I. | 0 | 3,3 U/ml (Aktivität = 100%) | 3,0 U/ml (Aktivität = 100%) | Testmix 1 aus Test- |
| | 15 | 1,9 U/ml (Restaktivität= 59%) | 3,0 U/ml (Restaktivität= 100%) | kombination "Creatinin- |
| | 30 | 0,6 U/ml (Restaktivität= 18%) | 2,7 U/ml (Restaktivität= 91%) | PAP" (BM Nr.839 434) |
| | 60 | 0,2 U/ml (Restaktivität= 6%) | 2,5 U/ml (Restaktivität= 83%) | |
| II. | 0 | 3,3 U/ml (Aktivität = 100%) | 3,0 U/ml (Aktivität = 100%) | in 125 mmol/l |
| | 15 | 0,7 U/ml (Restaktivität= 21%) | 2,7 U/ml (Restaktivität= 91%) | Phosphatpuffer |
| | 30 | 0,0 U/ml (Restaktivität= 0%) | 2,7 U/ml (Restaktivität= 91%) | + Detergenz |
| | 60 | 0,0 U/ml (Restaktivität= 0%) | 2,1 U/ml (Restaktivität= 71%) | |

Die Enzyme wurden ,unter den in der Tabelle angegebenen Bedingungen inkubiert und anschließend eine Enzymbestimmung unter Verwendung der Testkombination "Harnstoff" (BM Best.-Nr. 124 770) durchgeführt.

## Tabelle II

Enzymbestimmung unter optimalen Bedingungen bei verschiedenen Temperaturen

Testbedingungen: 20 mmol/l Phosphat-Puffer, pH 8,0

1.=Creatinamidinohydrolase-Wildtyp: 1,05 mg Protein/ml (8,0 U/mg)
2.=Creatinamidinohydroalse-Mutante: 1,10 mg Protein/ml (8,7 U/mg)
(Aminosäure 109 = Val)

| Temp. | Creatinase-Restaktivität in % (Inkubation in min) | | | | | |
|---|---|---|---|---|---|---|
| | 1. (DSM 3143) | | | 2. (DSM 4105) | | |
| | 30min | 60 | 120 | 30min | 60 | 120 |
| 37°C | 100 | 86 | 80 | 100 | 96 | 90 |
| 42°C | 57 | 36 | 13 | 84 | 73 | 63 |
| 47°C | 1 | 0,4 | 0 | 42 | 2 | 0 |
| 52°C | 0 | - | - | 3 | - | - |

Die Enzyme wurden unter den angegebenen Testbedingungen inkubiert und anschließend eine Enzymbestimmung unter Verwendung der Testkombination "Harnstoff" (BM Best. Nr. 124 770) durchgeführt.

Das erfindungsgemäße Creatinamidinohydrolaseenzym, welches zusätzlich zu dieser Mutation noch eine weitere Aminosäuremutation trägt, besitzt bei ebenfalls fast gleicher Ausgangsenzymaktivität eine noch größere Stabilität im Vergleich zum Wildtypenzym (Beispiel 1, Tabelle III).

Es ist daher mit den erfindungsgemäßen Creatinamidinohydrolasemutanten möglich, einen durch die Detergenz-und Thermolabilität des Wildtypenzyms verursachten Enzymaktivitätsverlust bei der Creatininbestimmung zu vermeiden und solche Bestimmungen auch über längere Zeiträume als bisher möglich, zuverlässig durchzuführen. Aufgrund der verbesserten Eigenschaften ist auch eine Reduzierung der Enzymmenge im Creatinin-Test möglich.

Die folgenden Beispiele erläutern die Erfindung weiter.

**Beispiel 1**

Zellen von E.coli DSM 4105, E.coli DSM 4106 und, zum Vergleich E.coli DSM 3143 (DE 35 00 184 A1) wurden über Nacht in 1l-Fermentern angezogen.

Medium

Vollmedium (Hefe/Peptonextrakt)
0,4 % Glucose
100 mmol/l Phosphat-Puffer

Nach 15 minütiger Zentrifugation bei 8.000 rpm wurden die Zellen in einer French Press aufgeschlossen und die Creatinamidinohydrolase durch Fraktionierung über ein Molekularsieb (Sephacryl S 200) gereinigt.

Die erhaltenen Enzyme wurden unter den in Tab. III angegebenen Bedingungen inkubiert und anschließend eine Enzymbestimmung unter Verwendung der Testkombination "Harnstoff" (BM Best.-Nr. 124 770) durchgeführt.

Tab. III gibt das Stabilitätsverhalten der erfindungsgemäßen Creatinamidinohydrolasemutanten im Vergleich zum Wildtypenzym (DE 35 00 184 A1 - E.coli DSM 3143) wieder.

Tabelle III

Stabilität bei 37°C

Testbedingungen: Testmix 1 aus Testkombination "Creatinin-PAP" (BM Best.-Nr. 839 434)

Ausgangsaktivität: 12 U/ml (= 100 %)

| Creatinamidinohydrolase aus E.coli (kodierendes Plasmid) | Restaktivität nach 15 | 30 | 45 | 60 min (% der Ausgangsaktivität) |
|---|---|---|---|---|
| DSM 3143 (pBT 2a-1 DSM 3148P) | 69 | 51 | 39 | 27 |
| DSM 4105 (pBT 109 DSM 4108P) | 96 | 90 | 84 | 84 |
| DSM 4106 (pBT 119 DSM 4107P) | 102 | 102 | 102 | 102 |

Vergleich der Michaelis Konstanten (Km) bei 37°C

Testbedingungen: 0,1 mol/l Tris-HCl, pH 8,0 (optimale Testbedingungen)

| Creatinamidinohydrolase aus E.coli (kodierendes Plasmid) | Km, mmol/l |
|---|---|
| DSM 3143 (pBT 2a-1 DSM 3148P) | 25 |
| DSM 4105 (pBT 109 DSM 4108P) | 20 |
| DSM 4106 (pBT 119 DSM 4107P) | 16 |

**Beispiel 2**

Die stabile Creatinamidinohydrolasemutante aus dem Mikroorganismus E. coli, DSM 4105, der das Plasmid pBT 109 enthält, wurde aus einer 100 l Fermentation, wie in der DE 35 00 184 A1 beschrieben, isoliert. Es wurden 121 g Protein mit einer spezifischen Aktivität von 10,4 U/mg erhalten. Dies entspricht einer Ausbeute von 63 %.

**Patentansprüche**

**Patentansprüche für folgende Vertragsstaaten : AT, BE, CH, DE, FR, GB, IT, LI, LU, NL, SE**

**1.** Creatinamidinohydrolase, welche die Reaktion

Creatin + $H_2O$ → Sarcosin + Harnstoff

katalysiert, **dadurch gekennzeichnet,** daß in Position 109 der Aminosäuresequenz des Wildtypenzyms Alanin durch Valin ersetzt ist.

**2.** Creatinamidinohydrolase nach Anspruch 1, **dadurch gekennzeichnet,** daß zusätzlich eine oder mehrere weitere Aminosäuren des Wildtypenzyms mutiert sind.

**3.** Plasmid pBT 109, DSM 4108P, **dadurch gekennzeichnet,** daß es das Gen für eine Creatinamidinohydrolase nach Anspruch 1 enthält.

**4.** Plasmid pBT 119, DSM 4107P, **dadurch gekennzeichnet,** daß es ein Gen für eine Creatinamidinohydrolase nach Anspruch 2 enthält.

**5.** Mikroorganismus E. coli, DSM 4105, **dadurch gekennzeichnet,** daß er das Plasmid pBT 109 enthält.

**6.** Mikroorganismus E. coli, DSM 4106, **dadurch gekennzeichnet,** daß er das Plasmid pBT 119 enthält.

**7.** Mikroorganismus der Gattung E. coli oder Pseudomonas putida, **dadurch gekennzeichnet,** daß er konstitutiv eine Creatinamidinohydrolase gemäß den Ansprüchen 1 bis 2 bildet.

8. Verfahren zur Herstellung des Enzyms nach Anspruch 1, **dadurch gekennzeichnet,** daß man nach bekannten, gentechnologischen Methoden in einem geeigneten Expressionssystem eine rekombinante DNA zur Expression bringt, welche ein Creatinamidinohydrolasegen enthält, das in Position 326 des natürlichen Gens anstelle von C das Desoxyribonukleotid T enthält.

9. Verfahren nach Anspruch 8, **dadurch gekennzeichnet,** daß man als rekombinante DNA das Plasmid pBT 109, DSM 4108P exprimiert.

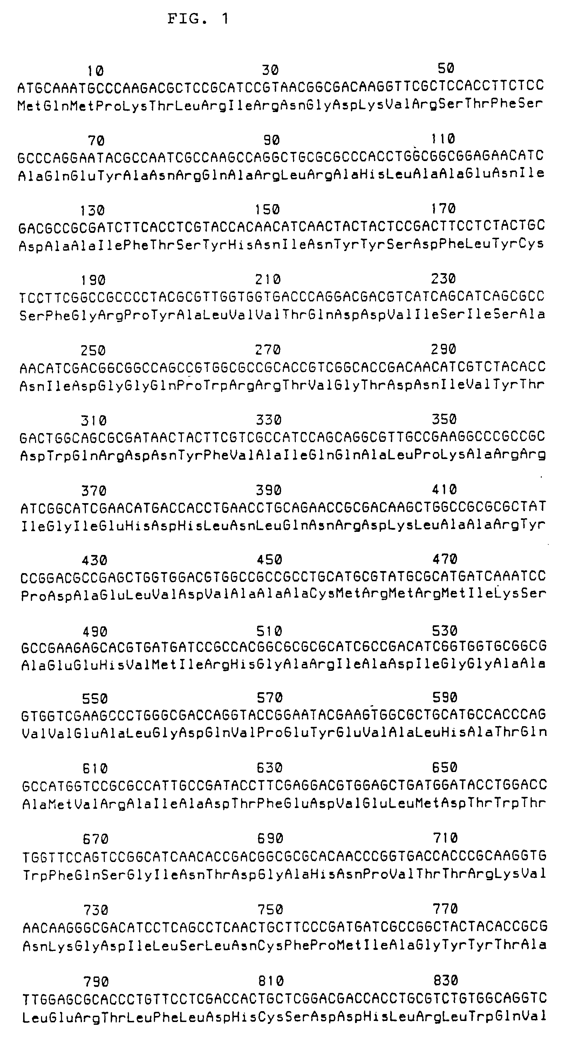

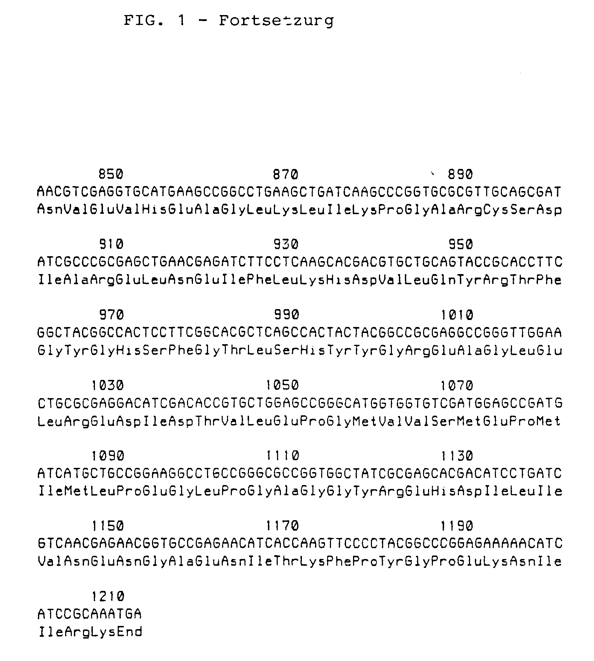

10. Verfahren nach Anspruch 8, **dadurch gekennzeichnet,** daß man eine rekombinante DNA verwendet, welche die in Fig. 1 dargestellte Sequenz oder ein nach dem genetischen Code für die gleiche Aminosäuresequenz kodierendes Äquivalent davon enthält

11. Verfahren zur Herstellung des Enzyms nach Anspruch 2, **dadurch gekennzeichnet,** daß man nach bekannten gentechnologischen Methoden in einem geeigneten Expressionssystem eine rekombinante DNA zur Expression bringt, welche ein Creatinamidinohydrolasegen enthält, welches zusätzlich zu der Mutation der Base C in T an der Position 326 des natürlichen Gens eine weitere Mutation enthält, die einen weiteren Aminosäureaustausch im Enzym bewirkt.

12. Verfahren nach Anspruch 11, **dadurch gekennzeichnet,** daß man ein rekombinantes Creatinamidinohydrolasegen exprimiert, welches in Position 1063 eine Mutation der Base G in A enthält.

13. Verfahren nach Anspruch 12, **dadurch gekennzeichnet,** daß man als rekombinante DNA das Plasmid pBT 119, DSM 4107P exprimiert.

14. Verfahren nach einem der Ansprüche 8 bis 13, **dadurch gekennzeichnet,** daß man als Expressionssystem einen Mikroorganismus der Gattung E. coli oder Pseudomonas putida verwendet.

15. Verfahren nach Anspruch 14, **dadurch gekennzeichnet,** daß man E. coli ED 8654, DSM 2102 verwendet.

16. Verfahren nach Anspruch 14, **dadurch gekennzeichnet,** daß man Pseudomonas putida, DSM 2106 verwendet.

17. Verfahren nach Anspruch 8 und 9, **dadurch gekennzeichnet,** daß man E. coli, DSM 4105 züchtet.

18. Verfahren nach den Ansprüchen 11, 12 und 13, **dadurch gekennzeichnet,** daß man E. coli, DSM 4106 züchtet.

19. Verwendung eines Enzyms nach Anspruch 1 und 2 zur Bestimmung des Creatiningehalts im Serum, Plasma oder Harn.

**Patentansprüche für folgende Vertragsstaaten : ES, GR**

1. Verfahren zur Herstellung von Creatinamidinohydrolase, welche die Reaktion

   Creatin + $H_2O$ → Sarcosin + Harnstoff

   katalysiert und in Position 109 der Aminosäuresequenz des Wildtypenzyms Valin anstelle von Alanin enthält, **dadurch gekennzeichnet,** daß man nach bekannten gentechnologischen Methoden in einem geeigneten Expressionssystem eine rekombinante DNA zur Expression bringt, welche ein Creatinamidinohydrolasegen enthält, das in Position 326 des natürlichen Gens anstelle von C das Desoxyribonukleotid T enthält.

2. Verfahren nach Anspruch 1,
   **dadurch gekennzeichnet,**
   daß man als rekombinante DNA das Plasmid pBT 109, DSM 4108P exprimiert.

3. Verfahren nach Anspruch 1,
**dadurch gekennzeichnet,**
daß man eine rekombinante DNA verwendet, welche die in Fig. 1 dargestellte Sequenz oder ein nach dem genetischen Code für die gleiche Aminosäuresequenz kodierendes Äquivalent davon enthält.

4. Verfahren nach einem der Ansprüche 1 bis 3,
**dadurch gekennzeichnet,**
daß man ein Creatinamidinohydrolasegen exprimiert, welches zusätzlich zu der Mutation der Base C in T an der Position 326 des natürlichen Gens eine weitere Mutation enthält, die einen weiteren Aminosäureaustausch im Enzym bewirkt.

5. Verfahren nach Anspruch 4,
**dadurch gekennzeichnet,**
daß man ein rekombinantes Creatinamidinohydrolasegen exprimiert, welches in Position 1063 eine Mutation der Base G in A enthält.

6. Verfahren nach Anspruch 5,
**dadurch gekennzeichnet,**
daß man als rekombinante DNA das Plasmid pBT 119, DSM 4107P exprimiert.

7. Verfahren nach einem der Ansprüche 1 bis 6,
**dadurch gekennzeichnet,**
daß man als Expressionssystem einen Mikroorganismus der Gattung E.coli oder Pseudomonas putida verwendet.

8. Verfahren nach Anspruch 7,
**dadurch gekennzeichnet,**
daß man E.coli ED 8654, DSM 2102 verwendet.

9. Verfahren nach Anspruch 7,
**dadurch gekennzeichnet,**
daß man Pseudomonas putida, DSM 2106 verwendet.

10. Verfahren nach Anspruch 1 oder 2,
**dadurch gekennzeichnet,**
daß man E.coli, DSM 4105 züchtet.

11. Verfahren nach den Ansprüchen 4, 5 oder 6,
**dadurch gekennzeichnet,**
daß man E.coli, DSM 4106 züchtet.

12. Verwendung eines Enzyms nach Anspruch 1 oder 2 zur Bestimmung des Creatiningehalts im Serum, Plasma oder Harn.

**Claims**
**Claims for the following Contracting States : AT, BE, CH, DE, FR, GB, IT, LI, LU, NL, SE**

1. Creatine amidinohydrolase, which catalyses the reaction:

creatine + $H_2O \rightarrow$ sarcosine + urea

characterised in that, in position 109 of an amino acid sequence of the wild type enzyme, alanine is replaced by valine.

2. Creatine amidinohydrolase according to claim 1, characterised in that, in addition, one or more further amino acids of the wild type enzyme are mutated.

**3.** Plasmid pBT 109, DSM 4108P, characterised in that it contains the gene for a creatine amidinohydrolase according to claim 1.

**4.** Plasmid pBT 119, DSM 4107P, characterised in that it contains a gene for a creatine amidinohydrolase according to claim 2.

**5.** Micro-organism E. coli, DSM 4105, characterised in that it contains the plasmid pBT 109.

**6.** Micro-organism E. coli, DSM 4106, characterised in that it contains the plasmid pBT 119.

**7.** Micro-organism of the genus E. coli or Pseudomonas putida, characterised in that it constitutively forms a creatine amidinohydrolase according to claims 1 to 2.

**8.** Process for the production of the enzyme according to claim 1, characterised in that, according to known gene-technological methods, into a suitable expression system, one brings a recombinant DNA to expression which contains a creatine amidinohydrolase gene which, in position 326 of the natural gene, contains the desoxyribonucleotide T instead of C.

**9.** Process according to claim 8, characterised in that, as recombinant DNA, one expresses the plasmid pBT 109, DSM 4108P.

**10.** Process according to claim 8, characterised in that one uses a recombinant DNA which contains the sequence illustrated in Fig. 1 or an equivalent thereof coding according to the genetic code for the same amino acid sequence.

**11.** Process for the production of the enzyme according to claim 2, characterised in that, according to known gene technological methods, in a suitable expression system, one brings a recombinant DNA to expression which contains a creatine amidinohydrolase gene which, in addition to the mutation of the base C into T on position 326 of the natural gene, contains a further mutation which brings about a further amino acid exchange in the enzyme.

**12.** Process according to claim 11, characterised in that one expresses a recombinant creatine amidinohydrolase gene which contains a mutation of the base G into A in position 1063.

**13.** Process according to claim 12, characterised in that one expresses the plasmid pBT 119, DSM 4107P as recombinant DNA.

**14.** Process according to one of claims 8 to 13, characterised in that, as expression system, one uses a micro-organism of the genus E. coli or Pseudomonas putida.

**15.** Process according to claim 14, characterised in that one uses E. coli ED 8654, DSM 2102.

**16.** Process according to claim 14, characterised in that one uses Pseudomonas putida, DSM 2106.

**17.** Process according to claim 8 and 9, characterised in that one cultures E. coli, DSM 4105.

**18.** Process according to claims 11, 12 and 13, characterised in that one cultures E. coli, DSM 4106.

**19.** Use of an enzyme according to claim 1 and 2 for the determination of the creatinine content in serum, plasma or urine.

**Claims for the following Contracting States : ES, GR**

**1.** Process for the production of creatine amidinohydrolase which catalyses the reaction

creatine + $H_2O \rightarrow$ sarcosine + urea

and contains valine instead of alanine in position 109 of the amino acid sequence of the wild type

enzyme, characterised in that, according to known gene-technological methods, in a suitable expression system one brings to expression a recombinant DNA which contains a creatine amidinohydrolase gene which, in position 326 of the natural gene, contains desoxyribonucleotide T instead of C.

**2.** Process according to claim 1, characterised in that, as recombinant DNA, one expresses the plasmid pBT 109, DSM 4108P.

**3.** Process according to claim 1, characterised in that one uses a recombinant DNA which contains the sequence illustrated in Fig. 1 or an equivalent thereof coding according to the genetic code for the same amino acid sequence.

**4.** Process according to one of claims 1 to 3, characterised in that one expresses a creatine amidinohydrolase which, in addition to the mutation of the base C into T on position 326 of the natural gene, contains a further mutation which brings about a further amino acid exchange in the enzyme.

**5.** Process according to claim 4, characterised in that one expresses a recombinant creatine amidinohydrolase gene which contains a mutation of the base G into A in position 1063.

**6.** Process according to claim 5, characterised in that, as recombinant DNA, one expresses the plasmid pBT 119, DSM 4107P.

**7.** Process according to one of claims 1 to 6, characterised in that, as expression system, one uses a micro-organism of the genus E. coli or Pseudomonas putida.

**8.** Process according to claim 7, characterised in that one uses E. coli ED 8654, DSM 2102.

**9.** Process according to claim 7, characterised in that one uses Pseudomonas putida, DSM 2106.

**10.** Process according to claim 1 or 2, characterised in that one cultures E. coli, DSM 4105.

**11.** Process according to claims 4, 5 or 6, characterised in that one cultures E. coli, DSM 4106.

**12.** Use of an enzyme according to claim 1 or 2 for the determination of the creatinine content in serum, plasma or urine.

**Revendications**
**Revendications pour les Etats contractants suivants : AT, BE, CH, DE, FR, GB, IT, LI, LU, NL, SE**

**1.** Créatinamidinohydrolase qui catalyse la réaction

créatine + $H_2O$ → sarcosine + urée

caractérisée en ce qu'en position 109 de la séquence des acides aminés de l'enzyme de type sauvage l'alanine est remplacée par la valine.

**2.** Créatinamidinohydrolase selon la revendication 1, caractérisée en ce qu'en plus un ou plusieurs autres acides aminés de l'enzyme de type sauvage sont mutés.

**3.** Plasmide pBT 109, DSM 4108P, caractérisé en ce qu'il contient le gène d'une créatinamidinohydrolase selon la revendication 1.

**4.** Plasmide pBT 119, DSM 4107P, caractérisé en ce qu'il contient un gène d'une créatinamidinohydrolase selon la revendication 2.

**5.** Micro-organisme E. coli, DSM 4105, caractérisé en ce qu'il contient le plasmide pBT 109.

**6.** Micro-organisme E. coli, DSM 4106, caractérisé en ce qu'il contient le plasmide pBT 119.

7. Micro-organisme du genre E. coli ou Pseudomonas putida, caractérisé en ce qu'il forme de manière constitutive une créatinamidinohydrolase selon les revendications 1 et 2.

8. Procédé de préparation de l'enzyme selon la revendication 1, caractérisé en ce que, par des méthodes de génie génétique connues, l'on fait s'exprimer dans un système d'expression approprié un ADN recombiné qui contient un gène de créatinamidinohydrolase qui contient, en position 326 du gène naturel, le désoxyribonucléotide T au lieu de C.

9. Procédé selon la revendication 8, caractérisé en ce que l'on exprime, comme ADN recombiné, le plasmide pBT 109, DSM 4108P.

10. Procédé selon la revendication 8, caractérisé en ce que l'on utilise un ADN recombiné qui contient la séquence représentée sur la figure 1 ou un équivalent de celle-ci qui code la même séquence d'acides aminés selon le code génétique.

11. Procédé de préparation de l'enzyme selon la revendication 2, caractérisé en ce que, par des méthodes de génie génétique connues, l'on fait s'exprimer dans un système d'expression approprié un ADN recombiné qui contient un gène de créatinamidinohydrolase qui contient, en plus de la mutation de la base C en T en position 326 du gène naturel, une autre mutation qui entraîne un autre échange d'acides aminés dans l'enzyme.

12. Procédé selon la revendication 11, caractérisé en ce que l'on exprime un gène de créatinamidinohydrolase recombiné qui contient en position 1063 une mutation de la base G en A.

13. Procédé selon la revendication 12, caractérisé en ce que l'on exprime comme ADN recombiné le plasmide pBT 119, DSM 4107P.

14. Procédé selon l'une des revendications 8 à 13, caractérisé en ce que l'on utilise comme système d'expression un micro-organisme du genre E. coli ou Pseudomonas putida.

15. Procédé selon la revendication 14, caractérisé en ce que l'on utilise E. coli ED 8654, DSM 2102.

16. Procédé selon la revendication 14, caractérisé en ce que l'on utilise Pseudomonas putida, DSM 2106.

17. Procédé selon les revendications 8 et 9, caractérisé en ce que l'on cultive E. coli, DSM 4105.

18. Procédé selon les revendications 11, 12 et 13, caractérisé en ce que l'on cultive E. coli, DSM 4106.

19. Utilisation d'une enzyme selon les revendications 1 et 2 pour la détermination de la teneur en créatinine du sérum, du plasma ou de l'urine.

**Revendications pour les Etats contractants suivants : ES, GR**

1. Procédé de préparation de la créatinamidinohydrolase qui catalyse la réaction

créatine + $H_2O \rightarrow$ sarcosine + urée

et qui contient en position 109 de la séquence des acides aminés de l'enzyme de type sauvage la valine à la place de l'alanine, caractérisé en ce que, par des méthodes de génie génétique connues, l'on fait s'exprimer dans un système d'expression approprié un ADN recombiné qui contient un gène de créatinamidinohydrolase qui contient, en position 326 du gène naturel, le désoxyribonucléotide T au lieu de C.

2. Procédé selon la revendication 1, caractérisé en ce que l'on exprime, comme ADN recombiné, le plasmide pBT 109, DSM 4108P.

3. Procédé selon la revendication 1, caractérisé en ce que l'on utilise un ADN recombiné qui contient la séquence représentée sur la figure 1 ou un équivalent de celle-ci qui code la même séquence d'acides

aminés selon le code génétique.

4. Procédé selon l'une des revendications 1 à 3, caractérisé en ce que l'on exprime un gène de créatinamidinohydrolase qui contient, en plus de la mutation de la base C en T en position 326 du gène naturel, une autre mutation qui entraîne un autre échange d'acides aminés dans l'enzyme.

5. Procédé selon la revendication 4, caractérisé en ce que l'on exprime un gène de créatinamidinohydrolase recombiné qui contient en position 1063 une mutation de la base G en A.

6. Procédé selon la revendication 5, caractérisé en ce que l'on exprime comme ADN recombiné le plasmide pBT 119, DSM 4107P.

7. Procédé selon l'une des revendications 1 à 6, caractérisé en ce que l'on utilise comme système d'expression un micro-organisme du genre E. coli ou Pseudomonas putida.

8. Procédé selon la revendication 7, caractérisé en ce que l'on utilise E. coli ED 8654, DSM 2102.

9. Procédé selon la revendication 7, caractérisé en ce que l'on utilise Pseudomonas putida, DSM 2106.

10. Procédé selon la revendication 1 ou 2, caractérisé en ce que l'on cultive E. coli, DSM 4105.

11. Procédé selon la revendication 4, 5 ou 6, caractérisé en ce que l'on cultive E. coli, DSM 4106.

12. Utilisation d'une enzyme selon la revendication 1 ou 2 pour la détermination de la teneur en créatinine du sérum, du plasma ou de l'urine.

FIG. 1

```
        10                  30                  50
ATGCAAATGCCCAAGACGCTCCGCATCCGTAACGGCGACAAGGTTCGCTCCACCTTCTCC
MetGlnMetProLysThrLeuArgIleArgAsnGlyAspLysValArgSerThrPheSer

        70                  90                 110
GCCCAGGAATACGCCAATCGCCAAGCCAGGCTGCGCGCCCACCTGGCGGCGGAGAACATC
AlaGlnGluTyrAlaAsnArgGlnAlaArgLeuArgAlaHisLeuAlaAlaGluAsnIle

       130                 150                 170
GACGCCGCGATCTTCACCTCGTACCACAACATCAACTACTACTCCGACTTCCTCTACTGC
AspAlaAlaIlePheThrSerTyrHisAsnIleAsnTyrTyrSerAspPheLeuTyrCys

       190                 210                 230
TCCTTCGGCCGCCCCTACGCGTTGGTGGTGACCCAGGACGACGTCATCAGCATCAGCGCC
SerPheGlyArgProTyrAlaLeuValValThrGlnAspAspValIleSerIleSerAla

       250                 270                 290
AACATCGACGGCGGCCAGCCGTGGCGCCGCACCGTCGGCACCGACAACATCGTCTACACC
AsnIleAspGlyGlyGlnProTrpArgArgThrValGlyThrAspAsnIleValTyrThr

       310                 330                 350
GACTGGCAGCGCGATAACTACTTCGTCGCCATCCAGCAGGCGTTGCCGAAGGCCCGCCGC
AspTrpGlnArgAspAsnTyrPheValAlaIleGlnGlnAlaLeuProLysAlaArgArg

       370                 390                 410
ATCGGCATCGAACATGACCACCTGAACCTGCAGAACCGCGACAAGCTGGCCGCGCGCTAT
IleGlyIleGluHisAspHisLeuAsnLeuGlnAsnArgAspLysLeuAlaAlaArgTyr

       430                 450                 470
CCGGACGCCGAGCTGGTGGACGTGGCCGCCGCCTGCATGCGTATGCGCATGATCAAATCC
ProAspAlaGluLeuValAspValAlaAlaAlaCysMetArgMetArgMetIleLysSer

       490                 510                 530
GCCGAAGAGCACGTGATGATCCGCCACGGCGCGCGCATCGCCGACATCGGTGGTGCGGCG
AlaGluGluHisValMetIleArgHisGlyAlaArgIleAlaAspIleGlyGlyAlaAla

       550                 570                 590
GTGGTCGAAGCCCTGGGCGACCAGGTACCGGAATACGAAGTGGCGCTGCATGCCACCCAG
ValValGluAlaLeuGlyAspGlnValProGluTyrGluValAlaLeuHisAlaThrGln

       610                 630                 650
GCCATGGTCCGCGCCATTGCCGATACCTTCGAGGACGTGGAGCTGATGGATACCTGGACC
AlaMetValArgAlaIleAlaAspThrPheGluAspValGluLeuMetAspThrTrpThr

       670                 690                 710
TGGTTCCAGTCCGGCATCAACACCGACGGCGCGCACAACCCGGTGACCACCCGCAAGGTG
TrpPheGlnSerGlyIleAsnThrAspGlyAlaHisAsnProValThrThrArgLysVal

       730                 750                 770
AACAAGGGCGACATCCTCAGCCTCAACTGCTTCCCGATGATCGCCGGCTACTACACCGCG
AsnLysGlyAspIleLeuSerLeuAsnCysPheProMetIleAlaGlyTyrTyrThrAla

       790                 810                 830
TTGGAGCGCACCCTGTTCCTCGACCACTGCTCGGACGACCACCTGCGTCTGTGGCAGGTC
LeuGluArgThrLeuPheLeuAspHisCysSerAspAspHisLeuArgLeuTrpGlnVal
```

FIG. 1 - Fortsetzurg

```
        850                     870                     890
AACGTCGAGGTGCATGAAGCCGGCCTGAAGCTGATCAAGCCCGGTGCGCGTTGCAGCGAT
AsnValGluValHisGluAlaGlyLeuLysLeuIleLysProGlyAlaArgCysSerAsp

        910                     930                     950
ATCGCCCGCGAGCTGAACGAGATCTTCCTCAAGCACGACGTGCTGCAGTACCGCACCTTC
IleAlaArgGluLeuAsnGluIlePheLeuLysHisAspValLeuGlnTyrArgThrPhe

        970                     990                    1010
GGCTACGGCCACTCCTTCGGCACGCTCAGCCACTACTACGGCCGCGAGGCCGGGTTGGAA
GlyTyrGlyHisSerPheGlyThrLeuSerHisTyrTyrGlyArgGluAlaGlyLeuGlu

       1030                    1050                    1070
CTGCGCGAGGACATCGACACCGTGCTGGAGCCGGGCATGGTGGTGTCGATGGAGCCGATG
LeuArgGluAspIleAspThrValLeuGluProGlyMetValValSerMetGluProMet

       1090                    1110                    1130
ATCATGCTGCCGGAAGGCCTGCCGGGCGCCGGTGGCTATCGCGAGCACGACATCCTGATC
IleMetLeuProGluGlyLeuProGlyAlaGlyGlyTyrArgGluHisAspIleLeuIle

       1150                    1170                    1190
GTCAACGAGAACGGTGCCGAGAACATCACCAAGTTCCCCTACGGCCCGGAGAAAAACATC
ValAsnGluAsnGlyAlaGluAsnIleThrLysPheProTyrGlyProGluLysAsnIle

       1210
ATCCGCAAATGA
IleArgLysEnd
```